# EUROPÄISCHE PATENTSCHRIFT

(11) **EP 3 522 973 B1**
(45) Veröffentlichungstag und Bekanntmachung des Hinweises auf die Patenterteilung: **26.11.2025**
(21) Anmeldenummer: 17780417.6
(22) Anmeldetag: 06.10.2017
(51) Int. Cl.: A61M 39/00, A61J 1/14, A61J 1/20, A61M 39/08, A61J 1/10, A61M 39/20

(54) **KONNEKTOR FÜR EIN EINE FLÜSSIGKEIT ENTHALTENDES MEDIZINISCHES PACKMITTEL**
CONNECTOR FOR A MEDICAL PACKAGING CONTAINING A LIQUID
CONNECTEUR POUR UN EMBALLAGE MÉDICAL CONTENANT UN LIQUIDE

(30) Priorität: 08.10.2016 DE 102016012059
(43) Veröffentlichungstag der Anmeldung: 14.08.2019
(73) Patentinhaber: Fresenius Kabi Deutschland GmbH, 61352 Bad Homburg (DE)
(72) Erfinder: BRANDENBURGER, Torsten, 61203 Reichelsheim (DE); SCHWERER, Stefan, 61197 Florstadt (DE)
(74) Vertreter: Fresenius Kabi Deutschland GmbH
(86) Internationale Anmeldenummer: PCT/EP2017/075525
(87) Internationale Veröffentlichungsnummer: WO 2018/065596

(56) Entgegenhaltungen:
- WO-A1-03/013972
- WO-A1-2016/156242
- DE-A1- 102008 060 864
- DE-A1- 19 500 459
- JP-U- 3 004 402
- US-A1- 2005 137 566
- US-A1- 2008 262 466

## Beschreibung

### Gebiet der Erfindung

Die Erfindung betrifft einen Konnektor für ein eine Flüssigkeit enthaltendes medizinisches Packmittel.

Insbesondere betrifft die Erfindung einen ein Septum umfassenden Konnektor, welcher mit einem eine medizinische Flüssigkeit enthaltenden Beutel verbindbar bzw. verbunden ist.

### Hintergrund der Erfindung

Ein Septum umfassende Konnektoren zum Befüllen oder Entleeren von medizinischen Packmitteln sind bekannt. Insbesondere gibt es Infusions-, Transfusions- oder Enteralbeutel, die einen Konnektor mit einem Septum umfassen, das mit einem Spike oder einer Nadel durchstoßen werden kann. Beispielsweise kann dem Beutel über eine mit einer Nadel ausgestattete Spritze ein Wirkstoff zugeführt werden. Über einen mit dem Spike verbundenen Schlauch kann der Beutel entleert werden.

Die Offenlegungsschrift WO 2004/084793 A1 (Fresenius Kabi Deutschland GmbH) zeigt eine medizinische Verpackung. In einer Ausführungsform umfasst ein Beutel für eine medizinische Flüssigkeit zwei Konnektoren. Ein Konnektor ist z.B. zum Zuspritzen eines Wirkstoffs vorgesehen. Ein weiterer Konnektor ist zum Entnehmen des Beutelinhalts vorgesehen. Die Konnektoren umfassen jeweils ein Septum, welches mit einer abbrechbaren Kappe überdeckt ist. Die Dokumente WO 2016/156242 A1 und JP 3 004402 U zeigen Konnektoren und Septa aus dem Stand der Technik.

### Aufgabe der Erfindung

Vor diesem Hintergrund liegt der Erfindung die Aufgabe zugrunde, einen Konnektor bereit zu stellen, der sich flexibler einsetzen lässt.

### Zusammenfassung der Erfindung

Die Aufgabe der Erfindung wird durch einen Konnektor nach Anspruch 1, durch ein mit einem Konnektor versehenes medizinisches Packmittel nach Anspruch 13 sowie durch ein Septum nach Anspruch 14 gelöst.

Bevorzugte Ausführungsformen und Weiterbildungen der Erfindung sind dem Gegenstand der abhängigen Ansprüche, der Beschreibung sowie den Figuren zu entnehmen.

Die Erfindung betrifft zunächst einen Konnektor für ein eine Flüssigkeit enthaltendes medizinisches Packmittel.

Das medizinische Packmittel umfasst einen Behälter, in dem die medizinische Flüssigkeit gelagert ist. Der Konnektor dient insbesondere dem Befüllen, dem Zuführen bzw. Ergänzen einer Flüssigkeit (z.B. eines Wirkstoffs) und/oder dem Entleeren des Behälters. Der Behälter des Packmittels ist insbesondere als Beutel oder Flasche, z.B. als Kunststoffflasche, ausgebildet.

Der Konnektor umfasst ein Unterteil, welches ein Verbindungsstück für einen Behälter des medizinischen Packmittels und einen Durchgang aufweist. Das Unterteil ist insbesondere zumindest teilweise röhrenförmig ausgebildet und über das Verbindungsstück mit dem Behälter verbunden, vorzugsweise verklebt oder verschweißt. Über den Durchgang kann Flüssigkeit in den Konnektor gelangen.

In einer Ausgestaltung ist das Verbindungsstück des Unterteils im Wesentlichen schiffchenförmig ausgebildet. Dadurch kann mit einem als Beutel ausgebildeten Behälter eine fluiddichte und auch mechanisch stabile Verbindung hergestellt werden.

Der Durchgang ist mit einem Septum verschlossen. Unter einem Septum wird ein selbstschließendes, wiederverschließbares Elastomerelement verstanden, welches mit einem Spike und/oder auch mit einer Nadel durchstoßen werden kann, um Flüssigkeit zu entnehmen oder zuzuführen. Nach dem Herausziehen des Spikes und/oder der Nadel verschließt sich das Septum selbstständig.

Als Elastomermaterial kann beispielsweise Polyisopren verwendet werden.

Weiter umfasst der Konnektor ein Oberteil, welches mit einer abbrechbaren Kappe versehen ist, die das Septum überdeckt. Das Septum ist also erst nach Abbrechen der Kappe zugänglich. Die Kappe ermöglicht eine leichte Erkennung, ob der Konnektor bereits verwendet wurde. Weiter kann durch die Kappe das Septum steril gehalten werden.

Die Kappe kann beispielsweise als abschnittsweise flaches Griffstück ausgebildet sein.

Das Oberteil und das Unterteil sind vorzugsweise als Spritzgussbauteile ausgebildet. Vorzugsweise ist bzw. wird das Oberteil auf das Unterteil aufgesetzt.

Insbesondere sind das Oberteil und das Unterteil mittels einer formschlüssigen Verbindung miteinander verbunden. Bei einer Ausführungsform der Erfindung kann hierbei das Oberteil auf das Unterteil aufgeprellt werden. Bei einer alternativen oder ergänzenden Ausführungsform sind das Oberteil und das Unterteil stoffschlüssig verbunden, insbesondere verschweißt oder verklebt. Bei einer weiteren Ausführungsform der Erfindung sind Oberteil und Unterteil einstückig ausgebildet. Es versteht sich, dass im Sinne der Erfindung sich der Durchgang auch abschnittsweise durch das Oberteil erstrecken kann.

Die Kappe ist vorzugsweise zusammen mit dem Oberteil einstückig ausgebildet, wobei insbesondere durch eine ringförmige Reduzierung der Materialdicke zwischen Kappe und Oberteil eine Sollbruchlinie ausgebildet ist. Das Oberteil dient insbesondere der Aufnahme der Kappe.

Weiter kann, wie es bei einer Ausführungsform der Erfindung vorgesehen ist, dass Septum formschlüssig zwischen dem Oberteil und dem Unterteil eingeschlossen sein.

Gemäß der Erfindung umfasst das Septum eine Mehrzahl von Anschlüssen für einen Spike und/oder für eine Nadel. Die Anschlüsse sind dazu ausgebildet und geeignet von einem Spike, insbesondere zur Flüssigkeitsentnahme, durchstoßen zu werden. Sie können aber auch durch eine Nadel durchstoßen werden. Die Anschlüsse sind an der Oberseite des Septums als Erhebungen ausgebildet.

Durch das Vorhandensein mehrerer Anschlüsse kann der Konnektor flexibler verwendet werden. Insbesondere kann der Konnektor sowohl zur Flüssigkeitszufuhr als auch zur -entnahme eingesetzt werden. Auf einen zweiten Konnektor am Packmittel kann, wie es bei einer Ausführungsform der Erfindung vorgesehen ist, verzichtet werden.

Zudem wird durch die Mehrzahl von Anschlüssen eine mehrfache Entnahme mit einem Spike oder mehreren Spikes ermöglicht, ohne dass der gleiche Anschluss mehrfach verwendet werden muss.

Insbesondere umfasst das Septum zumindest drei Anschlüsse. Bei einer Ausführungsform umfasst das Septum zumindest drei Anschlüsse für einen Spike. Die Anschlüsse für einen Spike umfassen vorzugsweise eine erste Führung, die z.B. als Schlitz oder als zwei gekreuzte Schlitze ausgebildet ist. Die Schlitze erleichtern oder ermöglichen das Einstechen eines Spikes.

Bei einer Weiterbildung der Erfindung ist die Oberseite des Septums, welche die Anschlüsse aufweist, nach dem Abbrechen der Kappe für ein Abwischen der Anschlüsse zugänglich.

Hierzu umfasst das Septum vorzugsweise keine derart tiefen Einbuchtungen, die ein Abwischen verhindern würden. Weiter sollte ein angrenzender Rand des Oberteils allenfalls geringfügig höher liegen als die angrenzende Oberseite des Septums, insbesondere maximal 1 mm höher.

Vorzugsweise fluchtet die Oberseite des Septums im Wesentlichen mit einer angrenzenden Sollbruchlinie für die Kappe oder die Oberseite des Septums überragt die angrenzende Sollbruchlinie.

Die Anschlüsse des Septums können so leicht abgewischt werden, was trotz einer vorzugsweise vorhandenen Sterilität der Anschlüsse vorgeschrieben sein kann.

Die höchste Stelle der Anschlüsse liegt vorzugsweise maximal 1 mm unter der an das Septum angrenzenden Oberseite der Kappe. Insbesondere liegt die höchste Stelle der Anschlüsse mit der angrenzenden Oberseite der Kappe auf einer Ebene oder darüber. In einer Ausführungsform der Erfindung sind die Anschlüsse an ihrer Oberseite als kuppelförmige Erhebungen ausgebildet. Die Anschlüsse sind dadurch einfacher zugänglich, insbesondere bei einem möglichen Desinfizieren der Anschlüsse mittels Abwischen. Weiter können die Anschlüsse sich zumindest abschnittsweise um die Einstechstelle ersteckende Ringe oder Ringabschnitte, insbesondere ausbildet als Nut oder Steg, umfassen. Dadurch kann die Einstichstelle von dem Benutzer besser erkannt werden. Insbesondere in Kombination mit den Kreuzschlitzen kann die Einstichstelle noch besser erkannt werden.

Bei einer bevorzugten Ausführungsform der Erfindung ist ein zwischen der Kappe und dem Septum vorhandener Zwischenraum durch die Kappe hermetisch abgedichtet. Das Septum kann so sterilisiert werden oder steril bereitgestellt sein und bleibt auch bei Handhabung des mit dem Konnektor versehenen Packmittels steril. Eine hermetische Abdichtung lässt sich insbesondere dadurch bereitstellen, dass die Sollbruchlinie zwischen Oberteil und Kappe keine Öffnungen aufweist.

Bei einer Weiterbildung der Erfindung verjüngt sich der Querschnitt des Durchgangs vom Septum her in Richtung des Verbindungsstücks zumindest abschnittsweise. Insbesondere verjüngt bzw. verjüngen sich der Innendurchmesser und/oder der Außerdurchmesser, vorzugsweise sowohl Innendurchmesser als auch Außendurchmesser, in Richtung des Verbindungsstücks.

So kann auf einfache Weise ein Konnektor bereitgestellt werden, welcher für mehrere Anschlüsse, insbesondere mehrere Anschlüsse für jeweils einen Spike, groß genug ist.

Vorzugsweise hat das Septum einen nach dem Abbrechen der Kappe zugänglichen Durchmesser von mehr als 10 mm, besonders bevorzugt von mehr als 12 mm. Bei einer nicht kreiszylindrischen Form wird hierunter der größte Durchmesser verstanden.

Bei einer Ausführungsform der Erfindung ist das Septum mittels eines Formschlusselementes zwischen dem Oberteil und dem Unterteil fixiert. Insbesondere ist ein Formschlusselement vorgesehen, welches das Septum randseitig radial fixiert. Dies kann ein randseitig umlaufender Steg sein. Insbesondere umfasst das Septum ein randseitig umlaufendes T-förmiges Formschlusselement. Aufgrund des in radialer Richtung vorhandenen Formschlusses kann das Septum nicht durch Krafteinwirkung in axialer Richtung herausgedrückt werden.

Bei einer weiteren Ausführungsform der Erfindung umfasst das Septum auf seiner Unterseite jeweils unterhalb eines Anschlusses angeordnete röhrchenförmige Führungen. Diese dienen insbesondere als, vorzugsweise zweite, Führung für jeweils einen Spike. Die Unterseite der röhrchenförmigen Führungen liegt insbesondere tiefer als eine Unterseite des Formschlusselements. Dadurch kann eine verbesserte Führung für einen Spike bereitgestellt werden. Die Unterseite der röhrchenförmigen Führungen kann den untersten Bereich des Septums bilden. In einer Ausgestaltung geht die Unterseite der röhrchenförmigen Führungen bündig in eine Unterseite eines gegenüber dem Formschlusselement innenliegenden ringförmigen Abschnitts des Septums über. Dieser ringförmige Abschnitt kann an einer Innenseite des Durchgangs in dem Konnektor anliegen und dabei eine verbesserte Positionierung des Septums bewirken.

Die Erfindung betrifft des Weiteren ein medizinisches Packmittel mit einem Behälter für eine Flüssigkeit, welcher insbesondere als Infusions-, Transfusions- oder Enteralbeutel oder als eine medizinische Flüssigkeit enthaltende Flasche ausgebildet ist. Das medizinische Packmittel umfasst zumindest eine Ausführungsform des vorstehend beschriebenen erfindungsgemäßen Konnektors, welcher über das Verbindungsstück mit dem Behälter des medizinischen Packmittels verbunden ist. Insbesondere ist das Verbindungsstück mit dem Behälter, der insbesondere als Beutel oder Flasche ausgebildet ist, verklebt oder verschweißt. Weiterhin liegt im Bereich der Erfindung auch ein Septum für eine Ausführungsform des vorstehend beschriebenen Konnektors und/oder des vorstehend beschriebenen Packmittels. Das Septum umfasst eine Mehrzahl von Anschlüssen für einen Spike und/oder für eine Nadel. Die Anschlüsse sind als kuppelförmige Erhebungen ausgebildet.

### Kurzbeschreibung der Zeichnungen

Der Gegenstand der Erfindung soll im Folgenden Bezug nehmend auf ein Ausführungsbeispiel anhand der Zeichnungen Fig. 1 bis Fig. 9 näher erläutert werden.
Fig. 1 ist eine perspektivische Ansicht eines Ausführungsbeispiels eines erfindungsgemäßen Konnektors.
Fig. 2 ist eine Explosionsdarstellung des in Fig. 1 gezeigten Konnektors.
Fig. 3 ist eine Seitenansicht und Fig. 4 eine Schnittansicht des Konnektors.
Fig. 5 und Fig. 6 sind perspektivische Ansichten des Septums auf seine Oberseite (Fig. 5) und seine Unterseite (Fig. 6), welches Teil des zuvor dargestellten Konnektors ist.
Fig. 7 ist eine Draufsicht auf die Oberseite des Septums und
Fig. 8 eine Schnittdarstellung entlang der Linie A-A in Fig. 7.
Fig. 9 zeigt eine schematische Ansicht eines mit einem Konnektor versehenen medizinischen Packmittels.

### Detaillierte Beschreibung der Zeichnungen

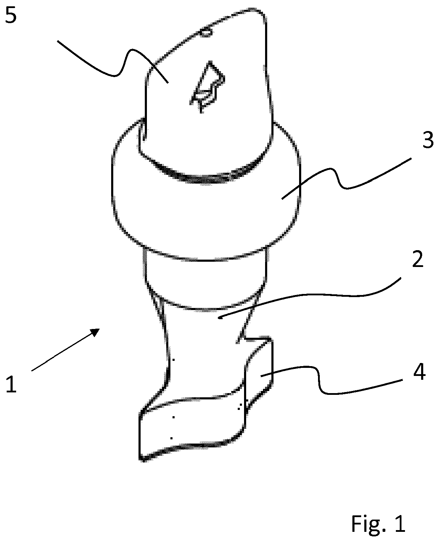

Fig. 1 ist eine perspektivische Ansicht eines Ausführungsbeispiels eines erfindungsgemäßen Konnektors 1. Der Konnektor 1 umfasst ein Unterteil 2 sowie ein auf das Unterteil 2 aufgesetztes Oberteil 3. Das Unterteil 2 umfasst an einem unteren Ende ein Verbindungsstück 4 für einen Behälter 21 des medizinischen Packmittels 20.

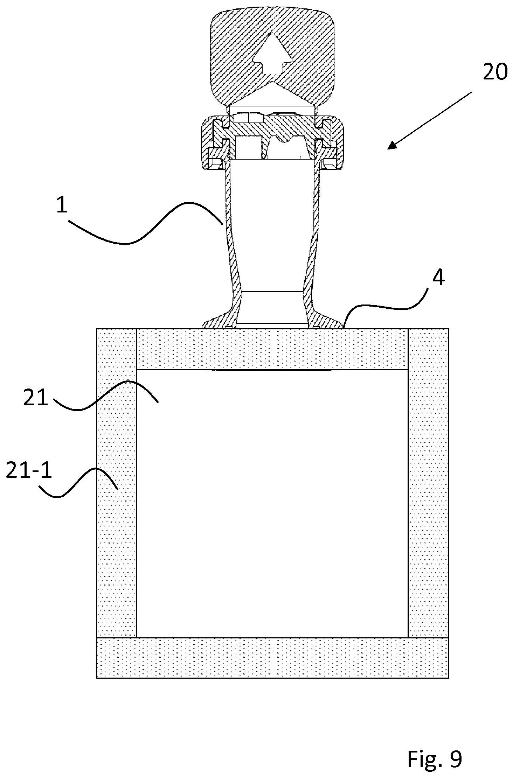

Das Verbindungsstück 4 ist in diesem Ausführungsbeispiel nicht kreiszylindrisch, sondern schiffchenförmig ausgebildet, was z.B. das Anbringen eines als Beutel ausgebildeten Behälters 21 erleichtert. Das Verbindungsstück 4 wird dabei in die den Rand des Beutels bildende Schweißnaht eingeschweißt (siehe dazu die Fig. 9). Vorzugsweise kann das Verbindungsstück 4 eine derart dicke Wandstärke besitzen, dass es z.B. nicht durch eine Nadel und/oder durch einen Spike durchstoßen werden kann.

Das Oberteil 3 hat einen größeren Außendurchmesser als das angrenzende Unterteil 2 im Bereich über dem Verbindungsstück 4 des Unterteils 3. Bei nicht kreiszylindrischer Form ist der jeweils größte Durchmesser der beiden zu vergleichenden Teile maßgeblich.

Weiter ist das Oberteil 3 mit einer abbrechbaren Kappe 5 versehen, die den oberen Abschnitt des Oberteils 3 bildet. Die Kappe 5 ist hier als flaches Griffstück ausgebildet, dessen Querschnitt sich von einer Kreisform zu einer plattenförmigen Ausgestaltung hin ändert. So kann die über eine Sollbruchlinie 10 verbundene Kappe 5 leicht von dem Oberteil 3 abgebrochen werden.

Die Kappe 5 hat im unteren Bereich mit kreisförmigem Querschnitt einen kleineren Durchmesser als der untere Abschnitt des Oberteils 3.

Die Kappe 5 stellt einen Originalitätsverschluss bereit. Nach dem Abbrechen der Kappe 5 ist die Oberseite 11 des Septums 6 zugänglich (hier nicht zu sehen).

In dem plattenförmigen Abschnitt der abbrechbaren Kappe 5 ist zudem eine als Pfeil ausgestaltete Öffnung eingebracht, welche den hier dargestellten Konnektor 1 als Entnahmekonnektor kennzeichnet.

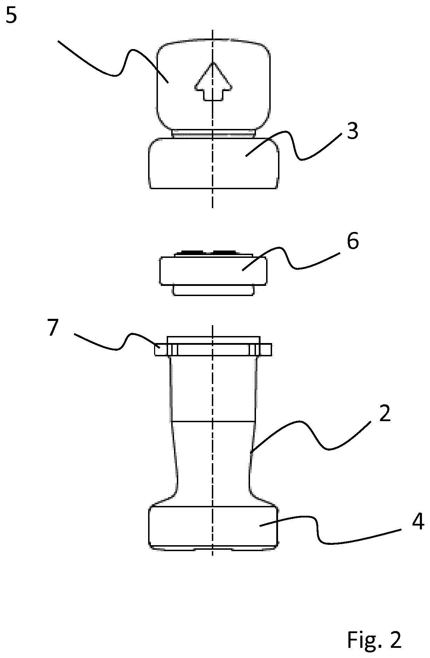

Fig. 2 ist eine Explosionsdarstellung des Konnektors 1, in welcher seine Einzelteile zu sehen sind. Der Konnektor 1 wird gebildet durch das Unterteil 2, das auf das Unterteil 2 aufgesetztes Oberteil 3 sowie das zwischen dem Oberteil 3 und dem Unterteil 2 eingesetzte Septum 6.

Das Unterteil 2 und das Oberteil 3 des Konnektors 1 sind hier mittels einer formschlüssigen Verbindung, beispielsweise einer Schnappverbindung, miteinander verbunden. In diesem Ausführungsbespiel umfasst das Unterteil 2 einen sich radial erstreckenden Kragen 7, auf welchen das Oberteil 3 aufgeprellt ist. So ist das Septum 6 formschlüssig und vorzugsweise auch klemmend zwischen dem Unterteil 2 und dem Oberteil 3 fixiert. Der Kragen 7 kann dabei in eine auf der Innenseite des Oberteils 3 eingebrachte Aussparung zum Eingriff kommen oder einen auf der Innenseite des Oberteils 3 angeordnete Vorsprung hintergreifen (nicht in Fig. 2 dargestellt).

Bei einer alternativen oder ergänzenden Ausführungsform (nicht dargestellt) sind das Oberteil 2 und das Unterteil 3 miteinander verschweißt oder verklebt oder als einstückiges Bauteil ausgebildet.

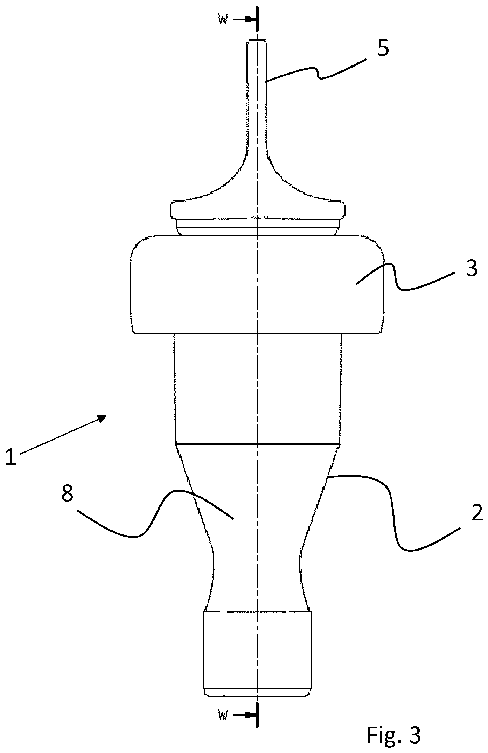

Fig. 3 ist eine Seitenansicht des Konnektors 1, in der insbesondere zu erkennen ist, dass die Kappe 5 als flaches Griffstück ausgebildet ist.

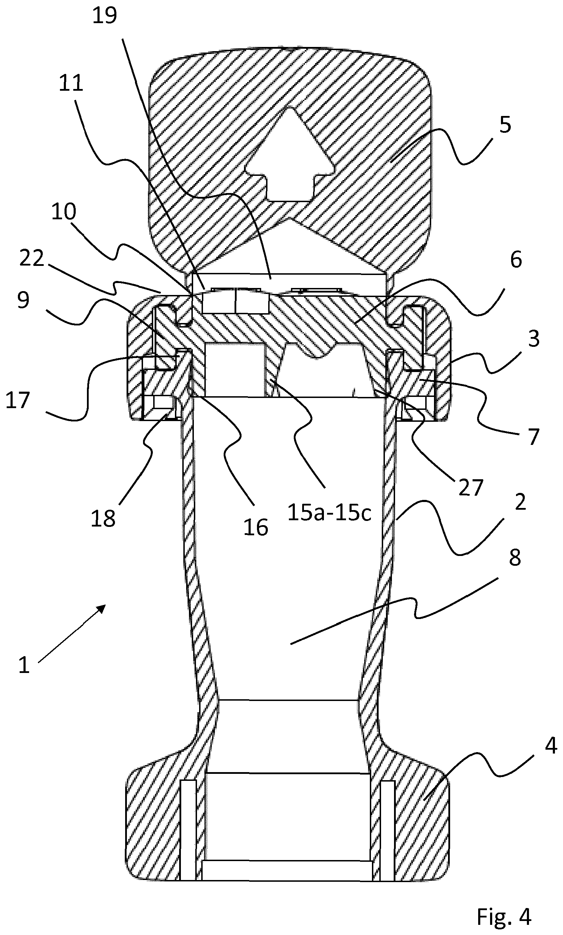

Fig. 4 ist eine Schnittdarstellung des Konnektors 1 entlang der Linie W-W gemäß der um 90° gedrehten Darstellung in Fig. 3.

Das Unterteil 2 weist einen Durchgang 8 auf, der sich vom Oberteil 3 ausgehend in Richtung des Verbindungsstücks 4 sowohl im Außendurchmesser als auch im Innendurchmesser verjüngt.

Der Durchgang 8 ist an seinem oberen Ende mit dem Septum 6 verschlossen.

Das Septum 6 ist in diesem Ausführungsbeispiel zwischen dem Oberteil 3 und dem Unterteil 2 eingesetzt. Das Septum 6 umfasst ein von seinem Rand ausgehendes T-förmiges Formschlusselement 9. Ein oberer Arm des T-förmigen Formschlusselements 9 sitzt in einer korrespondierenden Nut des Oberteils 3. Das Unterteil 2 umfasst an seiner Oberseite einen umlaufenden, sich in axialer Richtung erstreckenden Steg 17. Ein unterer Arm des T-förmigen Formschlusselements 9 hintergreift den Steg 17.Das elastische Septum 6 kann so nicht durch eine axiale Krafteinwirkung herausgedrückt werden.

Die Oberseite des Formschlusselementes 9 liegt unterhalb der Oberseite 11 des zentralen Abschnitts des Septums 6. So kann sich die Oberseite 22 des Oberteils 3 über das Formschlusselement 9 erstrecken und dabei bündig mit der angrenzenden Oberseite 11 des zentralen Abschnitts des Septums 6 fluchten.

Dadurch, dass die Oberseite 22 des Oberteils 3 und die Oberseite 11 des Septums 6 in etwa auf einer Ebene liegen, ist die Oberseite 11 des Septums 6 nach Abbrechen der Kappe 5 gut zugänglich und kann insbesondere leicht abgewischt werden.

Die Unterseite 16 des zentralen Abschnitts des Septums 6 liegt in diesem Ausführungsbeispiel tiefer als die Unterseite des T-förmigen Formschlusselementes 9.

Die Kappe 5 ist über eine ringförmige Sollbruchlinie 10 mit dem Oberteil 3 verbunden. Die Sollbruchlinie 10 fluchtet mit der angrenzenden Oberseite 11 des Septums 6. Weiter grenzt die Sollbruchlinie 10 unmittelbar an die nach Abbrechen der Kappe 5 zugängliche Oberfläche 11 des Septums 6 an.

Die Oberseite 11 des Septums 6 liegt nach dem Entfernen der Kappe 5 vollständig oder im Wesentlichen vollständig frei. Sowohl die drei Anschlüsse 12a, 12b, 12c als auch die zwischen den Anschlüssen 12a, 12b, 12c vorhandenen Flächen sind nicht überdeckt, z.B. von einem Gehäuseabschnitt des Konnektors 1.

Die Oberseite 11 des Septums 6 weist keine Vertiefungen auf. Nach dem Abbrechen der Kappe 5 ergibt sich so eine relativ glatte Fläche, die sich aus der Oberseite 11 des Septums 6 und der Oberseite 22 des Oberteils 3 zusammensetzt.

Zwischen der Kappe 5 und dem Septum 6 ist ein Zwischenraum 19 vorhanden, welcher vorzugsweise sterilisiert ist. Dadurch, dass die Sollbruchlinie 10 keine Öffnungen aufweist, ist der Zwischenraum 19 hermetisch abgedichtet.

In diesem Ausführungsbeispiel wird der Konnektor 1 zusammengesetzt, indem das Oberteil 3 auf das Unterteil 2 aufgeprellt wird. Nach dem Aufprellen greifen ein umlaufender Steg 18 des Oberteils 3 und/oder mehrere Rasthaken des Oberteils 3 (in den Figuren nicht dargestellt) unter den umlaufenden Kragen 7 des Unterteils 2. In einer alternativen oder ergänzenden Ausführungsform greift der Kragen 7 des Unterteils 2 in eine auf der Innenseite des Oberteils 3 eingebrachte Vertiefung ein (in den Figuren nicht dargestellt).

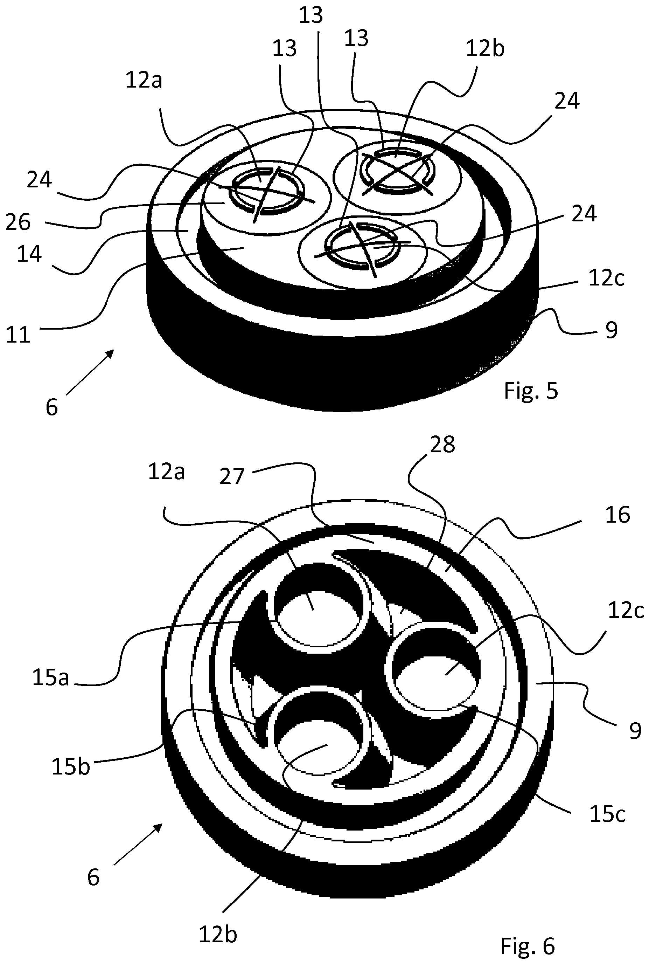

Fig. 5 ist eine perspektivische Detailansicht des Septums 6, in welcher die Oberseite 11 gut zu erkennen ist.

Das Septum 6 besteht aus einem Elastomermaterial, beispielsweise aus einem Polyisopren. Dieses kann z.B. peroxidisch oder mittels Schwefelbrücken vernetzt sein.

Zu erkennen ist das randseitig umlaufende T-förmige Formschlusselement 9, durch welches eine Nut 14 zwischen dem Formschlusselement 9 und der Oberseite 11 gebildet wird. In diese Nut 14 greift im montierten Zustand ein korrespondierender Steg des Oberteils 3.

Auf der Oberseite 11 sind in diesem Ausführungsbespiel drei Anschlüsse 12a-12c vorhanden.

Die Anschlüsse 12a-12c sind gleichmäßig auf dem Umfang des Septums verteilt. Der Winkel virtueller Verbindungslinien zum Mittelpunkt des Septums 6 beträgt somit zueinander jeweils etwa 120°. Die drei Anschlüsse 12a-12c bilden die Eckpunkte eines, vorzugsweise gleichseitigen, Dreiecks.

Die drei Anschlüsse 12a-12c sind in diesem Ausführungsbeispiel zur Aufnahme jeweils eines Spikes ausgebildet. Sie umfassen jeweils kreuzförmig ausgestaltete Schlitze 24. Die Schlitze 24 dienen als Führung für einen Spike. In diesem Ausführungsbeispiel durchdringen die Schlitze 24 das Septum 6 nicht vollständig. In einer anderen, hier nicht dargestellten Ausführungsform, laufen die Schlitze 24 durch die gesamte Höhe des Septums 6.

In einer hier nicht dargestellten Ausführungsform kann ein Anschluss auch nicht geschlitzt sein und zur Aufnahme einer Nadel dienen.

Die Anschlüsse 12a-12c sind in diesem Ausführungsbeispiel als kuppelförmige Erhebungen ausgebildet. Die Schlitze 24 der Anschlüsse 12a-12c kreuzen sich in einem jeweils abgeflachten Bereich der durch den jeweiligen Anschluss 12a-12c gebildeten Kuppel (siehe dazu auch die in Fig. 4 dargestellte Querschnittsansicht).

Die Einstechbereiche für die Spikes an der Oberseite 11 des Septums 6, welche bei den Anschlüssen 12a-12c durch die sich kreuzenden Schlitze 24 definiert sind, sind jeweils von einem ringförmig umlaufenden Steg 13 umschlossen. In diesem Ausführungsspiel bildet der Steg 13 gleichzeitig die Grenze des abgeflachten Bereichs der Kuppel. Die ringförmig umlaufenden Stege 13 sind jeweils an mehreren Stellen unterbrochen. Die Schlitze 24 erstrecken sich durch die Unterbrechungen bis in den abfallenden Bereich 26 der Kuppel, die durch den jeweiligen Anschluss 12a-12c gebildet wird.

Die hier dargestellte geometrische Ausgestaltung ermöglicht eine gute Zugänglichkeit und Handhabbarkeit der Einstechstellen des Septums 6.

Die zugängliche Oberfläche 11 des Septums 6 hat vorzugsweise zur Aufnahme der Anschlüsse 12a-12c einen Durchmesser von 10 mm bis 20 mm, bevorzugt von 12 mm bis 16 mm.

Die Gesamthöhe des Septums liegt vorzugsweise zwischen 5 mm und 20 mm.

Der Durchmesser des durch die ringförmigen Stege 13 eingegrenzten abgeflachten Bereiches der Kuppel beträgt vorzugsweise 2 mm bis 5 mm.

Fig. 6 ist eine perspektivische Ansicht, in welcher die Unterseite des Septums 6 gut zu erkennen ist.

Unterhalb der Anschlüsse 12a bis 12c erstrecken sich röhrchenförmige Führungen 15a bis 15c für jeweils einen Spike und/oder für eine Nadel.

Die röhrchenförmigen Führungen 15a bis 15c gehen randseitig in einen ringförmigen Abschnitt 27 des Septums 6 über, welcher die Unterseite 16 des Septums 6 bereitstellt. Im montierten Zustand liegt dieser ringförmige Bereich 27 an der Innenwand des Durchgangs 8 des Unterteils 2 an.

Vorzugsweise sind die röhrchenförmigen Führungen 15a bis 15c 1 mm bis 6 mm tief.

Der Zwischenraum 28 zwischen den röhrchenförmigen Führungen 15a bis 15c ist nicht mit Material gefüllt, so dass das Septum 6 im Bereich des Zwischenraums 28 in etwa dieselbe Dicke aufweist wie im Bereich der Anschlüsse 15a bis 15c.

Da im Bereich des Zwischenraums 28 sich keine Anschlüsse befinden, kann in einer hier nicht dargestellten Ausführungsform der Zwischenraum 28 auch bis zur Oberseite 16 mit Material aufgefüllt sein.

Im Bereich der Anschlüsse 15a bis 15c beträgt die Dicke des Septums 6 vorzugsweise 1 mm bis 5 mm.

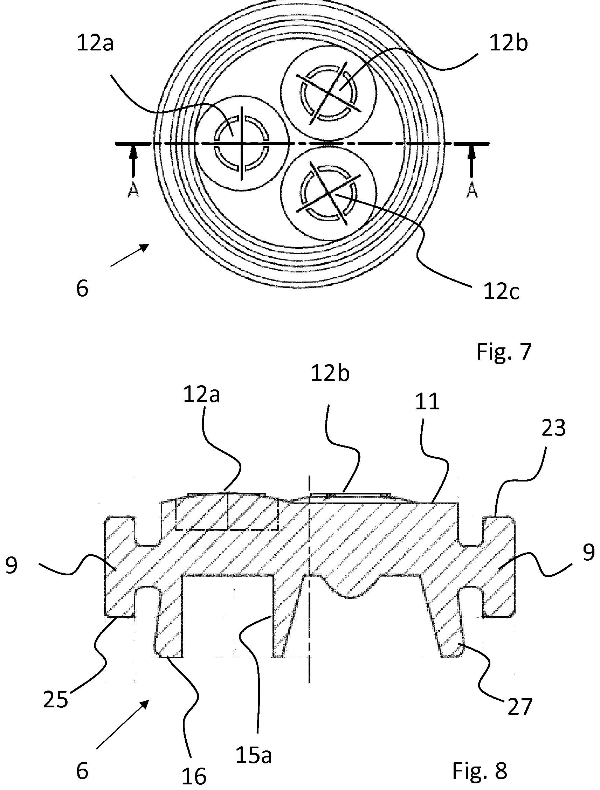

Fig. 7 ist eine Draufsicht auf die Oberseite des Septums 6 mit den drei Anschlüssen 12a bis 12c.

Fig. 8 ist eine Schnittdarstellung des Septums 6 entlang der Linie A-A aus Fig. 7.

Zu erkennen ist insbesondere, wie die kuppelförmig ausgebildeten Anschlüsse 12a bis 12c aus der angrenzenden Oberfläche 11 des Septums 6 herausragen.

Die Oberseite 23 des T-förmigen Formschlusselements 9 liegt tiefer als die durch die Oberseite 11 gebildete zugängliche Oberfläche des Septums 6.

Die Unterseite 25 des T-förmigen Formschlusselements 9 liegt höher als die Unterseite des ringförmigen Abschnitts 27, der die Unterseite 16 des Septums 6 bereitstellt. Die Unterseite des ringförmigen Abschnitts 27 geht bündig in die Unterseite der ringförmigen Führung 15a über.

Fig. 9 zeigt schematisch, wie der zuvor dargestellte Konnektor 1 mit dem Behälter 21 eines medizinischen Packmittels 20 verbunden ist.

In diesem Ausführungsbeispiel ist das Verbindungsstück 4 des Konnektors 1 mit einem als Beutel ausgebildeten Behälter 21 verschweißt. Das Verbindungsstück 4 ist in die Schweißnaht des Behälters 21 mit eingeschweißt.

Über den Konnektor 1 kann dem Behälter 21 Flüssigkeit sowohl entnommen als auch zugeführt werden. Über den Konnektor 1 kann der Behälter 21 auch befüllt werden, insbesondere bei noch nicht aufgesetztem Oberteil 3.

In dem gezeigten Beispiel besitzt der Behälter 21 lediglich einen einzelnen Konnektor 1. Vorzugsweise wird der Konnektor 1 nur zur Entnahme mittels eines Spikes verwendet. Der Konnektor 1 ist in diesem Fall ein Entnahmekonnektor.

Durch die Erfindung kann ein gut handhabbarer Konnektor für eine flexible Verwendung bereitgestellt werden.

### Bezugszeichenliste

- 1: Konnektor
- 2: Unterteil
- 3: Oberteil
- 4: Verbindungsstück
- 5: (Abbrechbare) Kappe
- 6: Septum
- 7: Kragen
- 8: Durchgang
- 9: Formschlusselement
- 10: Sollbruchlinie
- 11: Oberseite des Septums
- 12a-12c: Anschluss
- 13: Steg
- 14: Nut
- 15a-15c: Röhrchenförmige Führung
- 16: Unterseite der röhrchenförmigen Führung und/oder des Septums
- 17: Steg
- 18: Steg
- 19: Zwischenraum
- 20: Packmittel
- 21: Behälter
- 21-1: Schweißnaht des Behälters
- 22: Oberseite
- 23: Oberseite (des Formschlusselements 9)
- 24: Schlitz
- 25: Unterseite (des Formschlusselements 9)
- 26: Abfallender Bereich
- 27: Ringförmiger Abschnitt
- 28: Zwischenraum

## Patentansprüche

1. Konnektor (1) für ein eine Flüssigkeit enthaltendes medizinisches Packmittel (20), umfassend
ein Unterteil (2), welches ein Verbindungsstück (4) für einen Behälter (21) des medizinischen Packmittels (20) und einen Durchgang (8) aufweist,
ein Septum (6) nach einem der Ansprüche 14 bis 15, das den Durchgang (8) des Unterteils (8) verschließt, sowie
ein auf das Unterteil (2) aufgesetztes Oberteil (3), welches mit einer abbrechbaren Kappe (5) versehen ist, die das Septum (6) überdeckt.

2. Konnektor (1) nach dem vorstehenden Anspruch, **dadurch gekennzeichnet, dass** eine Oberseite (11) des Septums (6) nach dem Abbrechen der Kappe (5) für ein Abwischen der Anschlüsse (12a-12c) zugänglich ist.

3. Konnektor (1) nach einem der vorstehenden Ansprüche, **dadurch gekennzeichnet, dass** eine Oberseite (11) des Septums (6) im Wesentlichen mit einer angrenzenden Sollbruchlinie (10) für die Kappe (5) fluchtet oder dass eine Oberseite (11) des Septums (6) eine angrenzende Sollbruchlinie (10) für die Kappe (5) überragt.

4. Konnektor (1) nach einem der vorstehenden Ansprüche, **dadurch gekennzeichnet, dass** ein zwischen der Kappe (5) und dem Septum (6) vorhandener Zwischenraum (19) durch die Kappe (5) hermetisch abgedichtet und vorzugsweise steril bereitgestellt ist.

5. Konnektor (1) nach einem der vorstehenden Ansprüche, **dadurch gekennzeichnet, dass** sich Ringe (13) zumindest abschnittsweise um die Anschlüsse (12a-12c) erstecken.

6. Konnektor (1) nach einem der vorstehenden Ansprüche, **dadurch gekennzeichnet, dass** sich der Querschnitt des Durchgangs (8) in dem Unterteil (2) des Konnektors (1) von dem Septum (6) her in Richtung des Verbindungsstücks (4) zumindest abschnittsweise verjüngt.

7. Konnektor (1) nach einem der vorstehenden Ansprüche, **dadurch gekennzeichnet, dass** das Verbindungsstück (4) des Unterteils (2) im Wesentlichen schiffchenförmig ausgebildet ist.

8. Konnektor (1) nach einem der vorstehenden Ansprüche, **dadurch gekennzeichnet, dass** das Septum (1) einen nach dem Abbrechen der Kappe (5) zugänglichen Durchmesser von mehr als 10 mm, vorzugsweise von mehr als 12 mm, aufweist.

9. Konnektor (1) nach einem der vorstehenden Ansprüche, **dadurch gekennzeichnet, dass** das Septum (6) ein randseitig umlaufendes T-förmiges Formschlusselement (9) umfasst, welches zwischen dem Oberteil (3) und dem Unterteil (2) des Konnektors (1) fixiert ist.

10. Konnektor (1) nach einem der vorstehenden Ansprüche, **dadurch gekennzeichnet, dass** das Septum (6) auf seiner Unterseite unterhalb der Anschlüsse (12a-12c) angeordnete röhrchenförmige Führungen (15a-15c) aufweist.

11. Konnektor (1) nach einem der beiden vorstehenden Ansprüche, **dadurch gekennzeichnet, dass** eine Unterseite (16) der röhrchenförmigen Führungen (15a-15c) tiefer als eine Unterseite (25) des Formschlusselements (9) liegt.

12. Konnektor (1) nach einem der drei vorstehenden Ansprüche, **dadurch gekennzeichnet, dass** die Unterseite (16) der röhrchenförmigen Führungen (15a-15c) bündig in eine Unterseite eines gegenüber dem Formschlusselement (9) innenliegenden ringförmigen Abschnitts (27) des Septums (6) übergeht.

13. Medizinisches Packmittel (20) mit einem Behälter (21), insbesondere ausgebildet als Infusions-, Transfusions- oder Enteralbeutel oder als eine medizinische Flüssigkeit enthaltende Flasche, umfassend einen Konnektor (1) nach einem der vorstehenden Ansprüche 1 bis 12 oder ein Septum (6) nach einem der Ansprüche 14 bis 15.

14. Septum (6) für einen Konnektor (1) und/oder für einen Behälter (21) eines medizinisches Packmittels (20), wobei das Septum (6) eine Mehrzahl von Anschlüssen (12a-12c) für einen Spike umfasst, die zumindest abschnittsweise geschlitzt ausgebildet sind, und dadurch charakterisiert, dass die Anschlüsse (12a-12c) kuppelförmig ausgebildet sind.

15. Septum (6) nach vorstehendem Anspruch 14, **dadurch gekennzeichnet, dass** das Septum (6) auch mit einer Nadel durchstoßen werden kann, um Flüssigkeit zu entnehmen oder zuzuführen.

## Claims

1. A connector (1) for a medical package (20) containing a liquid, said connector comprising
a lower part (2), which has a connecting piece (4) for a container (21) of the medical package (20) and a passage (8),
a septum (6) according to one of claims 14 to 15, which closes the passage (8) of the lower part (8), and
an upper part (3) which is placed on the lower part (2) and which is provided with a cap (5) which can be broken off and which covers the septum (6).

2. The connector (1) according to the preceding claim, **characterised in that** an upper side (11) of the septum (6) is accessible for wiping the connections (12a-12c) after the cap (5) has been broken off.

3. The connector (1) according to one of the preceding claims, **characterised in that** an upper side (11) of the septum (6) is substantially aligned with an adjoining predetermined breaking line (10) for the cap (5) or **in that** an upper side (11) of the septum (6) projects beyond an adjoining predetermined breaking line (10) for the cap (5).

4. The connector (1) according to one of the preceding claims, **characterised in that** an intermediate space (19) present between the cap (5) and the septum (6) is hermetically sealed by the cap (5) and is preferably provided in a sterile state.

5. The connector (1) according to one of the preceding claims, **characterised in that** rings (13) extend at least in sections around the connections (12a-12c).

6. The connector (1) according to one of the preceding claims, **characterised in that** the cross-section of the passage (8) in the lower part (2) of the connector (1) tapers at least in sections from the septum (6) in the direction of the connecting piece (4).

7. The connector (1) according to one of the preceding claims, **characterised in that** the connecting piece (4) of the lower part (2) is substantially ship-shaped.

8. The connector (1) according to one of the preceding claims, **characterised in that** the septum (1) has a diameter of more than 10 mm, preferably more than 12 mm, which is accessible after the cap (5) has been broken off.

9. The connector (1) according to one of the preceding claims, **characterised in that** the septum (6) comprises a T-shaped form-fitting element (9) which runs around the edge and which is fixed between the upper part (3) and the lower part (2) of the connector (1).

10. The connector (1) according to one of the preceding claims, **characterised in that** the septum (6) has tubular guides (15a-15c) arranged on its underside below the connections (12a-12c).

11. The connector (1) according to one of the two preceding claims, **characterised in that** an underside (16) of the tubular guides (15a-15c) lies lower than an underside (25) of the form-fitting element (9).

12. The connector (1) according to one of the three preceding claims, **characterised in that** the underside (16) of the tubular guides (15a-15c) merges flush into an underside of an annular section (27) of the septum (6) lying internally with respect to the form-fitting element (9).

13. A medical package (20) with a container (21), in particular designed as an infusion, transfusion or enteral bag or as a bottle containing a medical liquid, comprising a connector (1) according to one of the preceding claims 1 to 12 or a septum (6) according to one of the claims 14 to 15.

14. A septum (6) for a connector (1) and/or for a container (21) of a medical package (20), wherein the septum (6) comprises a plurality of connections (12a-12c) for a spike, which are slotted at least in sections, and **characterised in that** the connections (12a-12c) are dome-shaped.

15. The septum (6) according to the preceding claim 14, **characterised in that** the septum (6) can also be pierced with a needle in order to remove or supply liquid.

## Revendications

1. Connecteur (1) pour un emballage médical (20) contenant un liquide, comprenant
une partie inférieure (2), laquelle présente une pièce de liaison (4) pour un récipient (21) de l'emballage médical (20) et un passage (8),
un septum (6) selon l'une des revendications 14 à 15, qui obture le passage (8) de la partie inférieure (8), ainsi que
une partie supérieure (3) placée sur la partie inférieure (2), laquelle est pourvue d'un capuchon (5) sécable qui recouvre le septum (6).

2. Connecteur (1) selon la revendication précédente, **caractérisé en ce qu'**une face supérieure (11) du septum (6) est accessible pour un essuyage des raccords (12a-12c) après la rupture du capuchon (5).

3. Connecteur (1) selon l'une des revendications précédentes, **caractérisé en ce qu'**une face supérieure (11) du septum (6) est sensiblement à fleur avec une ligne de rupture (10) adjacente pour le capuchon (5) ou **en ce qu'**une face supérieure (11) du septum (6) fait saillie d'une ligne de rupture (10) adjacente pour le capuchon (5).

4. Connecteur (1) selon l'une des revendications précédentes, **caractérisé en ce qu'**un espace intermédiaire (19) présent entre le capuchon (5) et le septum (6) est scellé hermétiquement par le capuchon (5) et est de préférence fourni de manière stérile.

5. Connecteur (1) selon l'une des revendications précédentes, **caractérisé en ce que** des anneaux (13) s'étendent au moins par sections autour des raccords (12a-12c).

6. Connecteur (1) selon l'une des revendications précédentes, **caractérisé en ce que** la section transversale du passage (8) dans la partie inférieure (2) du connecteur (1) se rétrécit au moins par sections à partir du septum (6) en direction de la pièce de liaison (4).

7. Connecteur (1) selon l'une des revendications précédentes, **caractérisé en ce que** la pièce de liaison (4) de la partie inférieure (2) est réalisée sensiblement en forme de nacelle.

8. Connecteur (1) selon l'une des revendications précédentes, **caractérisé en ce que** le septum (1) présente un diamètre accessible après rupture du capuchon (5) supérieur à 10 mm, de préférence supérieur à 12 mm.

9. Connecteur (1) selon l'une des revendications précédentes, **caractérisé en ce que** le septum (6) comprend un élément de complémentarité de forme (9) en forme de T qui fait le tour du bord, lequel élément qui est fixé entre la partie supérieure (3) et la partie inférieure (2) du connecteur (1).

10. Connecteur (1) selon l'une des revendications précédentes, **caractérisé en ce que** le septum (6) présente sur sa face inférieure des guides (15a-15c) de forme tubulaire agencés en dessous des raccords (12a-12c).

11. Connecteur (1) selon l'une des deux revendications précédentes, **caractérisé en ce qu'**une face inférieure (16) des guides (15a-15c) de forme tubulaire est située plus bas qu'une face inférieure (25) de l'élément de complémentarité de forme (9).

12. Connecteur (1) selon l'une des trois revendications précédentes, **caractérisé en ce que** la face inférieure (16) des guides (15a-15c) de forme tubulaire se prolonge en affleurement par une face inférieure d'une section (27) de forme annulaire du septum (6) située à l'intérieur par rapport à l'élément de complémentarité de forme (9).

13. Emballage médical (20) comportant un récipient (21), en particulier réalisé comme une poche de perfusion, de transfusion ou entérale ou comme un flacon contenant un liquide médical, comprenant un connecteur (1) selon l'une des revendications 1 à 12 précédentes ou un septum (6) selon l'une des revendications 14 à 15.

14. Septum (6) pour un connecteur (1) et/ou pour un récipient (21) d'un emballage médical (20), dans lequel le septum (6) comprend une pluralité de raccords (12a-12c) pour une pointe, qui sont réalisés avec des fentes au moins par sections, et **caractérisé en ce que** les raccords (12a-12c) sont réalisés en forme de coupole.

15. Septum (6) selon la revendication 14 précédente, **caractérisé en ce que** le septum (6) peut également être percé à l'aide d'une aiguille afin de prélever ou de fournir du liquide.
